# EUROPEAN PATENT APPLICATION

(11) **EP 1 852 056 A1**
(43) Date of publication of application: **07.11.2007**
(21) Application number: 07008424.9
(22) Date of filing: 25.04.2007
(51) Int. Cl.: A61B 1/12

(54) **Endoscope system**

(30) Priority: 01.05.2006 JP 2006127190
(71) Applicant: Fujinon Corporation, Saitama-shi, Saitama (JP)
(72) Inventor: Kawanishi, Tetsuya, Saitama-shi Saitama (JP)
(74) Representative: Klunker . Schmitt-Nilson . Hirsch

(57) **Abstract**

An endoscope system is provided and includes: a plurality of guide tubes that is pierced into a subject coelom swelled by supplying a pressurized gas; an endoscope inserted into one of the plurality of guide tubes; a treatment piece inserted into at least the other one of the plurality of guide tubes; an injection nozzle that intermittently inject a cleaning gas to an observation window at a front end of an inserting portion of the endoscope so as to flow along a surface of the observation window, the cleaning gas being the same as the pressurized gas; and a cleaning gas supply controller capable of setting an injection pressure and an injection time period of the cleaning gas under a condition that an injection amount of the cleaning gas becomes equal to or smaller than a leakage amount of the pressurized gas leaked from inside of the coelom.

## Description

### BACKGROUND OF THE INVENTION

### 1. Field of the Invention

The present invention relates to an endoscope system for forming a fluid curtain at an observation window provided at a front end of an endoscope of a celioscope or the like.

### 2. Description of Related Art

In a celioscope surgical operation, an operation or a treatment of ablation of an affected part of a tumor or the like in an inner wall of the coelom or the organ or the like, ablation of the organ, closure, hemostasis or the like is carried out, and therefore, a burden on a patient is not so serious as in celiotomy. In the celioscope surgical operation, an aeroperitonia gas is filled in the celiac coelom to considerably swell the celiac coelom. Further, a plurality of guide tubes comprising trocar or the like are inserted into the celiac coelom, a celioscope is inserted from one of the guide tubes to acquire an image of inside of the celiac coelom to carry out an observation.

Further, the affected part is treated by a treatment piece of a high frequency treatment piece of an electric surgical knife or the like in the celiac coelom swelled by the aeroperitonia gas while observing the image of inside of the celiac coelom. At this occasion, a cautery treatment is applied to the affected part by the treatment piece, when the cautery treatment is carried out, smoke, steam or the like is generated. Since the inside of the celiac coelom is a closed space, generated smoke, steam or the like stays at inside of the celiac coelom, and these are adhered to the observation window provided at the front end portion of the celioscope.

The observation window provided at the front end of the inserting portion of the celioscope is a portion constituting a front end of an observation optical system for taking an image of an inspected part, and therefore, when the smoke, steam or the like is adhered to the observation window, a clear field of view of the celioscope cannot be ensured. Hence, Patent Reference 1 discloses a constitution of restraining contamination from being adhered to an observation window of an endoscope. According to JP-A-2005-176908, a front end of an endoscope is provided with a fluid curtain start end side flow path for making a fluid flow along the observation window, and a fluid curtain finish end side flow path provided on a downstream side of a flow of the fluid after passing through the observation window. An aeroperitonia gas flows out from the fluid curtain start end side flow path, and the flowed aeroperitonia gas is sucked to the fluid curtain finish end side flow path. Thereby, a surface of the observation window is formed with a protection film by the fluid.

The invention of JP-A-2005-176908 achieves an extremely high effect in that the observation window can be restrained from being contaminated, and therefore, an always excellent observation field of view can be ensured in the observation by the endoscope or in applying a treatment thereby since the protection film by the fluid curtain is formed on the surface of the observation window. When the fluid curtain as the protection film is previously formed to be inline with the surface of the observation window, the observation window can be prevented from being contaminated beforehand, and therefore, the constitution is particularly effective in that it is not necessary to clean the observation window after contaminating the observation window as a posterior measure.

Here, CO₂ gas is mainly used as the aeroperitonia gas from a view point of protection of a patient. It is necessary to minimize a consumption amount of CO₂ gas from a view point of effecting an adverse influence on an environment. In this respect, according to Patent Reference 1, CO₂ gas forming the fluid curtain sucked from the fluid curtain finish end side flow path is returned again to the celiac coelom by circulating substantially a total amount thereof to return to the fluid curtain start end side flow path again, and therefore, CO₂ gas is restrained from being consumed wastefully. However, the consumption amount of CO₂ gas used as the fluid curtain is restrained by being circulated, and therefore, the apparatus tends to be large-sized in view of needing a filter apparatus, a sterilization system or the like for removing various viruses or the like from inside of the gas.

On the other hand, there are various factors of deteriorating the observation field of view of the endoscope, there is also conceivable a case in which the observation window cannot sufficiently be protected by only forming the above-described fluid curtain. Smoke, steam or the like is gradually filled at inside of the celiac coelom, and therefore, a speed of their own is not a high speed, and an energy provided to smoke, steam or the like is not so much strong. Therefore, in most of the cases, a function of protecting the observation window can sufficiently be achieved by the fluid curtain. However, otherwise, various treatments are applied at inside of the celiac coelom, and therefore, contaminants or the like scatter at a high speed depending on a kind of the treatment. At this occasion, when the function of protecting the observation window cannot sufficiently be achieved by the fluid curtain, it is necessary to ensure the observation field of view by separate means. That is, it is necessary to apply means for ensuring the observation field of view in correspondence with the kind of the treatment applied to the affected part.

### SUMMARY OF THE INVENTION

An object of an illustrative, non-limiting embodiment of the present invention is to provide an endoscope system in which an observation window of a front end of an endoscope is restrained from being contaminated and a consumption amount of CO₂ gas is restrained without making an apparatus large-sized. Another object is to provide an endoscope system ensuring an observation field of view without depending on a kind of a treatment.

According to an aspect of the invention, there is provided an endoscope system comprising: a plurality of guide tubes that is pierced into a subject coelom swelled by supplying a pressurized gas; an endoscope inserted into one of the plurality of guide tubes; a treatment piece inserted into at least the other one of the plurality of guide tubes; an injection nozzle that intermittently inject a cleaning gas to an observation window at a front end of an inserting portion of the endoscope so as to flow along a surface of the observation window, the cleaning gas being the same as the pressurized gas; and a cleaning gas supply controller capable of setting an injection pressure and an injection time period of the cleaning gas under a condition that an injection amount of the cleaning gas becomes equal to or smaller than a leakage amount of the pressurized gas leaked from inside of the coelom.

### BRIEF DESCRIPTION OF THE DRAWINGS

The features of the invention will appear more fully upon consideration of the exemplary embodiment of the invention, which are schematically set forth in the drawings, in which:
Fig. 1 is an outline constitution view of an endoscope system according to an exemplary embodiment of the invention;
Fig. 2 is an explanatory view of a front end of an endoscope;
Fig. 3 is an outline constitution view of gas supply controller; and
Figs. 4A and 4B illustrate time charts of an injection pressure and an injection time period.

### DETAILED DESCRIPTION OF THE EXEMPLARY EMBODIMENTS

Although the invention will be described below with reference to the exemplary embodiments thereof, the following exemplary embodiments and modifications do not restrict the invention.

According to an endoscope system of an exemplary embodiment of the invention, the observation field of view can be ensured without depending on the kind of the treatment and at the same time, the consumption amount of the aeroperitonia gas can be restrained without making the apparatus large-sized.

An exemplary embodiment of the invention will be explained in reference to the drawings as follows. According to an exemplary embodiment of the invention, an endoscope utilizing a celioscope will be explained, and therefore, the celioscope will be explained as a hard endoscope. Naturally, an exemplary embodiment of the invention is applicable also to various endoscopes other than the celioscope. However, although an exemplary embodiment of the invention is applied mainly to inside of the celiac coelom since an observation field of view of the endoscope is ensured at inside of a closed space, an exemplary embodiment of the invention is applicable to an arbitrary part so far as the closed space is a closed space other than inside of the celiac coelom.

According to a celioscope surgical operation, a treatment, an inspection or the like is carried out in a state of previously swelling a belly portion of a patient. Therefore, the belly portion is expanded by supplying an aeroperitonia gas as a pressurized gas to inside of the celiac coelom by an aeroperitonia apparatus. Thereby, safety can be ensured and ranges of operating a treatment piece and an endoscope can be ensured. Although as the aeroperitonia gas, it is preferable to use CO₂ gas from the view point of protection of the patient, the object of the invention can be achieved by also using other kind of a gas. However, it is a condition that the gas used for cleaning the observation window mentioned later is a gas the same as the aeroperitonia gas.

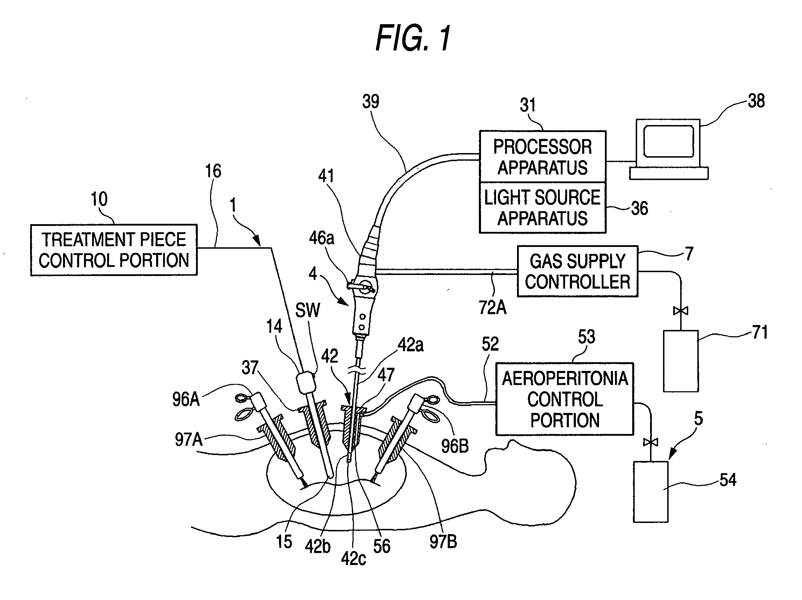

Referring to Fig. 1, an endoscope system of the embodiment includes a treatment piece 1 and a hard endoscope 4 and aeroperitonia apparatus 5. The treatment 1 includes a treatment piece controlling portion 10 and a treatment piece operating portion 14, the treatment piece operating portion 14 and the treatment piece controlling portion 10 are connected by a connecting cord 16. The hard endoscope 4 is connected to a processor apparatus 31 and a light source apparatus 36 by a universal cord 39, the processor apparatus 31 is connected to a monitor apparatus 38. The hard endoscope 4 is connected with a gas supply controller 7 by way of a gas sending tube 72A, the gas supply controller 7 is connected to a first CO₂ gas bomb 71. The aeroperitonia apparatus 5 includes an aeroperitonia tube 52 and an aeroperitonia control portion 53 and a second CO₂ gas bomb 54, the aeroperitonia tube 52, the aeroperitonia control portion 53 and the second CO₂ gas bomb 54 are connected in this order. 4 pieces of trocars are inserted to the belly portion of the patient as guide tubes as exemplified in Fig. 1. A first trocar 37 is inserted with the treatment piece 1 (an insertion cord 15 mentioned later), a second trocar 47 is inserted with the hard endoscope 4 (inserting portion 42 mentioned later). A third trocar 97A and a fourth trocar 97B are respectively inserted with treatment pieces 96A and 96B (forceps 96A and 96B in the drawing).

The treatment piece 1 is used for carrying out an operation or a treatment of ablation of an affect zone, ablation of the organ, hemostasis or the like at inside of the coelom as in a high frequency treatment piece, an ultrasonic solidification and incision apparatus of, for example, an electric surgical knife or the like. The treatment piece 1 is provided with the treatment piece operating portion 14 operated by an operator, by pressing down a switch SW provided to the treatment piece operating portion 14 by the operator, the inserting cord 15 of an electric surgical knife or the like is operated, and operation of an affected part or the like is carried out.

The hard endoscope 4 includes a main body operating portion 41 and an inserting portion 42, a hard portion 42a, an angle portion 42b, and a front end hard portion 42c are connected in this order from the main body operating portion 41. An amount of a length of most of the inserting portion 42 is occupied by the hard portion 42a in order to inspect inside of the celiac coelom. However, a soft endoscope can also be used, in that case, a potion of the hard portion 42a is constituted by a soft portion. The angle portion 42b is made to be able to be operated to bend in an up and down direction and a left and right direction by remote operation in order to direct the front end hard portion 42c in a desired direction. Therefore, the main body operating portion 41 is provided with an angle operating portion 46a, the angle portion 42b is bent by operation of the operator to control to direct the front end hard portion 42c to the desired direction.

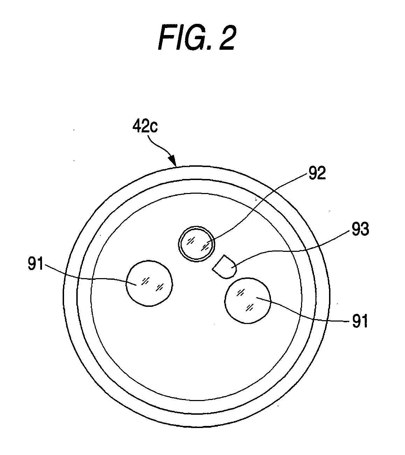

The front end hard portion 42c of the inserting portion 42 is provided with an observing portion for observing an inspected part. As shown by Fig. 2, the endoscope observing portion of the front end hard portion 42c is substantially constituted by including an illumination window 91 and an observation window 92 and an injection nozzle 93. The illumination window 91 is connected to a light guide, not illustrated, incorporated in the universal cord 39 and the light guide is connected to the light source apparatus 36. The light source apparatus 36 is a light source for supplying illuminating light, the illuminating light from the light source apparatus 36 is illuminated from the illumination window 91 to the inspected portion by way of the light guide. In the drawing, the illumination windows 91 are arranged at two portions to interpose the observation window 92.

The observation window 92 constitutes a front end of the observation optical system and an object lens, a solid-state imaging device are arranged at a poststage of inside of the front end hard portion 42c, although not illustrated. An image of the respective portion provided from the observation window 92 is focused to a position of the solid imaging device by the object lens. The solid-state imaging device generates an image signal by carrying out photoelectric conversion. The solid-state imaging device is connected with a signal line, not illustrated, connected to a signal line of inside of the universal cord 39, the signal line is connected to the processor apparatus 31. Therefore, the image signal generated by the solid-state imaging device is inputted to the processor apparatus 31, subjected to a signal processing by the processor apparatus 31, an image of observing the detected portion is acquired, and an image signal is outputted to the monitor apparatus 38. Further, the image of the detected portion is displayed on a screen.

When a surface of the observation window 92 is contaminated, in order to clean the surface, a cleaning solution or a cleaning gas is selectively injected from the injection nozzle 93. After cleaning the surface of the observation window 92, in order to remove liquid drops of the cleaning solution adhered to the surface, the cleaning gas is blown to remove liquid drops adhered to the observation window 92. Here, CO₂ gas is used as the cleaning gas. Therefore, at inside of the hard endoscope 4, a liquid sending path, not illustrated, constituting a flow path of the cleaning solution and an air sending tube path, not illustrated, constituting a flow path of CO₂ gas are joined (joined mainly at a vicinity of a front end portion of the front end hard portion 42c), and a tube path after having been joined is guided to the injection nozzle 93.

The main body operating portion 41 is attachably and detachably connected to and from the gas sending tube 72A connected to the gas supply controller 7. The gas supply controller 7 controls to supply CO₂ gas supplied from the first CO₂ gas bomb 71, and the CO₂ gas is guided from the gas sending tube 72A to the main body operating portion 41. The main body operating portion 41 is arranged with the gas sending tube path constituting the flow path of CO₂ gas to the injection nozzle 93, and therefore, by connecting the gas sending tube path and the gas sending tube 72A, the CO₂ gas of the first CO₂ gas bomb 71 can be guided to the injection nozzle 93, and the CO₂ gas can be injected.

The aeroperitonia apparatus 5 is an apparatus for supplying CO₂ gas to inside of the celiac coelom. In carrying out a treatment by using the treatment piece 1 or carrying out an inspection by using the hard endoscope 4, when the treatment is carried out by using the forceps 96A and 96B or the like, in order to ensure safety, further, it is necessary to make a movable range as wide as possible, and the celiac coelom is swelled by a gas. From a view point of protection of the patient, as the aeroperitonia gas, not air but CO₂ gas is used. Hence, CO₂ gas filled in the second CO₂ gas bomb 54 is supplied to inside of the celiac coelom by the control of the aeroperitonia control portion 53. Therefore, the second trocar 47 is provided with the gas path 56 constituting the flow path of CO₂ gas. By connecting the gas path 56 and the aeroperitonia tube 52, CO₂ gas of the second CO₂ gas bomb 54 is supplied to inside of the celiac coelom by passing the aeroperitonia tube 52 and the gas path 56 by the control of the aeroperitonia control portion 53.

As described above, a cleaning unit (cleaning by cleaning solution) of the observation window 92 is for cleaning the contaminant such that the contaminant is removed when a large amount of the contaminant is adhered to the observation window 92. However, when observation or treatment is carried out at inside of the celiac coelom, the operator does not grasp the hard endoscope 4 by the hand but mounts the hard endoscope 4 to a holder to be intent on operation of a treatment piece inserted from other trocar. Although the main body operating portion 41 of the hard endoscope 4 is provided with gas sending and water sending valves for sending cleaning water or sending CO₂ gas, the operator operates not the hard endoscope 4 but the other treatment piece, and therefore, it is difficult for the operator to operate the gas sending and water sending valves.

In order to reduce a frequency of cleaning by operating the gas sending and water sending valves, CO₂ gas as the cleansing gas is automatically injected from the injection nozzle 93 to be inline with the surface of the observation window 92. Thereby, the observation window 92 is cleaned without operating the gas sending and water sending valves by the operator. Since there is adopted a constitution capable of injecting CO₂ gas from the injection nozzle 93 for removing liquid drops of the cleaning solution, CO₂ gas can be injected from the injection nozzle 93 to be inline with the surface of the observation window 92.

Further, CO₂ gas is controlled to be injected intermittently. That is, 1 cycle is constituted by a predetermined time period, a time period of injecting CO₂ gas and a time period of stop injecting CO₂ gas are provided during 1 cycle. By cyclically carrying out the operation, the intermittent injection control operation is carried out. Here, there are a case of needing an injection pressure and a case of needing an injection time period for CO₂ gas injected to be inline with a surface of the observation window 92. The reason will be explained.

There are various factors of deteriorating the observation field of view by contaminating the observation window 92. First, one factor is smoke generated when a high frequency knife or the like is used, when the smoke is adhered to the observation window 92, the observation field of view is deteriorated. Further, inside of the celiac coelom is under a highly humid atmosphere, and therefore, when a treatment piece inserted to inside of the celiac coelom is heated, also a surrounding thereof is brought into a high temperature state, and there is a case in which steam is adhered to the observation window 92. Further, there is also a case in which a body fluid of the blood, the fat or the like is adhered to the observation window 92. Further, when an ultrasonic solidification and incision apparatus is used as the treatment piece 1, since ablation or solidification or the like of an affected part is carried out by a force of the ultrasonic vibration having a very high frequency in the ultrasonic solidification and incision apparatus, in a case of the tissue having much water, there is a case of bringing about steam explosion of scattering a large amount of mist. Then, mist scattered by steam explosion is adhered to the observation window 92.

Therefore, since there are the various factors of the contaminating various kinds of the observation windows 92, in order to clean the observation window 92, a method of dealing therewith differs in correspondence with kinds of respective treatments. In various kinds of factors for deteriorating the observation field of view, with regard to smoke generated when a cautery treatment is carried out, smoke is gradually filled at inside of the celiac coelom, and therefore, a speed of the smoke per se is not so high (that is, an inertia force provided to smoke, steam or the like is small). Then, although it is not necessary to blow CO₂ gas to the observation window 92 by a high speed in order to remove smoke, since smoke stays at inside of the celiac coelom, it is preferable to protect the observation window 92 as long as possible. Therefore, the injection time period of CO₂ gas is prolonged.

On the other hand, in order to remove mist adhered to the observation window 92 by steam explosion, although a long injection time period is not needed, it is necessary to blow CO₂ gas to the observation window 92 at high speed. Because mist generated by steam explosion is scattered at high speed, and therefore, an adhered strength of mist adhered to the observation window 92 is strong.

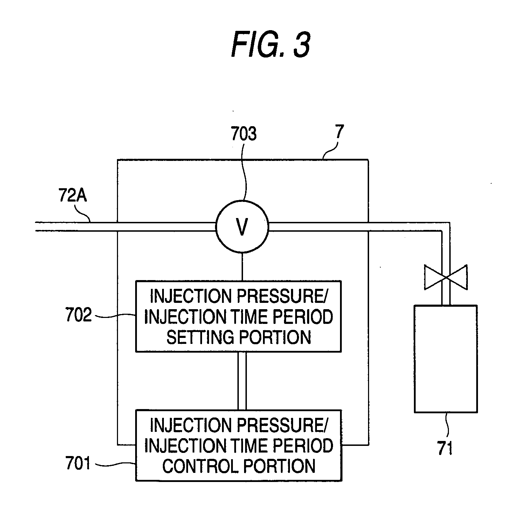

Hence, as shown by Fig. 3, the gas supply controller 7 is provided with an injection pressure/injection time period setting unit 701 to enable the operator to set arbitrary injection pressure or injection time period. Thereby, it can be set whether injection pressure is predominant or the injection time period is predominant in order to clean the observation window 92. However, when CO₂ gas is injected unlimitedly from the injection nozzle 93, inside of the celiac coelom is brought into a high pressure state, and therefore, it is necessary to limit the injection amount of CO₂ gas to be equal to or smaller than a predetermined amount. Hence, the injection pressure or the injection time period is set under a condition of being a limited amount or less. When the limited amount is previously determined, by setting either one of the injection pressure or the injection time period, other thereof are uniquely be determined. Therefore, either one of the injection pressure or the injection time period may be set. Further, a time period of 1 cycle for carrying out the intermittent operation is simultaneously set. In this case, although numerical values of various kinds of settings can directly be inputted to the injection pressure/injection time period setting unit 701, a constitution capable of selecting a mode of treatment may be adopted.

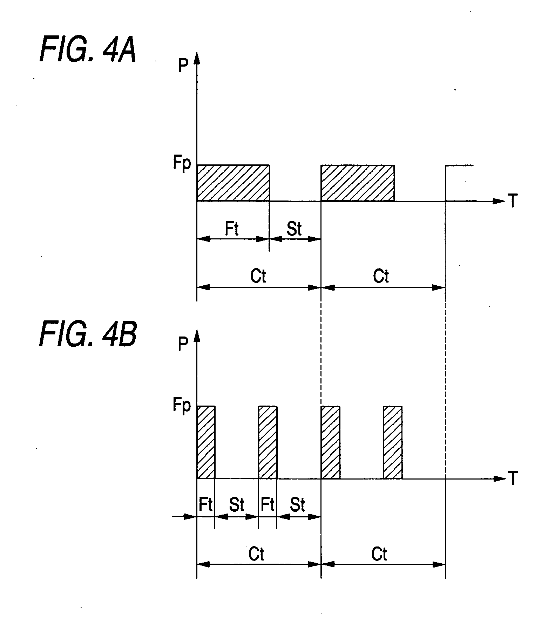

In the following, assume that 2 kinds of modes of a high frequency knife mode and an ultrasonic solidification and incision apparatus mode are previously set as treatment modes. The high frequency knife mode is a mode for removing smoke generated by the cautery treatment, and therefore, the high frequency knife mode is set with a long injection time period. On the other hand, the ultrasonic solidification and incision apparatus mode is a mode for removing mist generated by steam explosion, and therefore, the injection pressure is set to be high. Fig. 4 shows time charts at this occasion.

Further, although here, 2 kinds of the treatment modes are exemplified, the treatment modes may further be subdivided, a plurality of kinds of 3 kinds or more may be made to be able to be set, further, 1 kind of the treatment mode can be constituted. For example, when 3 kinds of treatment modes are prepared, combinations of the injection pressure and the injection time period can be set such that in a first treatment mode, the injection pressure is made to be high, the injection time period is made to be short, in a second treatment mode, the injection pressure is made to be middle, the injection time period is made to be middle, in a third treatment mode, the injection pressure is made to be low, the injection time period is made to be long or the like.

In Figs. 4A and 4B, the abscissa designates the injection time period (T), the ordinate designates the injection pressure (P). Fig. 4A shows a time chart of the high frequency knife mode, Fig. 4B shows a time chart of an ultrasonic solidification and incision apparatus mode. In Figs. 4A nd 4B, notation Ct is a time period of set 1 cycle. During 1 cycle, CO₂ gas is controlled to inject from the injection nozzle 93 by the injection pressure/injection time period in accordance with the treatment.

Fig. 4A shows the high frequency knife mode, and therefore, the injection time period is made to be predominant. That is, it is necessary to ensure the long injection time period. When the injection time period is ensured to be long, it is necessary to make the injection pressure low in proportion thereto. Because when the injecting amount is limited for avoiding inside of the celiac coelom from being brought into a high pressure state, in a case of ensuring the long injection time period, it is necessary to make the injection pressure low by that amount. In Fig. 4A, although CO₂ gas is injected once during the time period Ct of the set 1 cycle, the injection time period Ft is long, and a stop time period St is short. Further, the injection pressure Fp is made to be comparatively low.

Fig. 4B shows the ultrasonic solidification and incision apparatus mode, and therefore, high pressure injection is preferable. That is, the high injection pressure is ensured. As described above, when the injection pressure is made to be high, the injection time period is made to be short. In Fig. 4B, although CO₂ gas is injected twice during the time period Ct of set 1 cycle, the injection time period Ft is short, the stop time period St is long. Further, the injection pressure Fp is constituted by high value.

Although the injection pressures and the injection time periods respectively differ in the high frequency knife mode and the ultrasonic solidification and incision apparatus mode, a product of the injection pressure by the injection time period per 1 cycle in Figs. 4A and 4B, that is, the injecting amount of CO₂ gas (area in the drawing) is made to be equal. The injection is controlled such that the injection amount of CO₂ gas becomes equal to or smaller than the limit value in setting either of the modes.

CO₂ gas injected from the injection nozzle 93 is controlled to supply by injection pressure/injection time period controller 702 provided to the gas supply controller 7. The injection pressure/injection time period controller 702 determines the injection pressure and the injection time period in accordance with the selected mode. The injection pressure/injection time period controller 702 is connected with an electromagnetic proportional valve 703, based on the control of the injection pressure/injection time period control unit 702, the electromagnetic proportional valve 703 controls to open/close the valve and controls to make the flow path area variable. That is, when the control of prolonging the injection time period is carried out by the injection pressure/injection time period controller 702, the electromagnetic proportional valve 703 prolongs a time period of opening the valve in order to prolong the injection time period and narrows a valve opening degree in order to make the injection pressure low. On the other hand, when the control of making the injection pressure high is carried out, the electromagnetic proportional valve 703 increases the valve opening degree of the valve in order to make the injection pressure high, and makes the time period of opening the valve short in order to shorten the injection time period. By controlling the electromagnetic proportional valve 703, the injection time period and the injection pressure of CO₂ gas injected from the injection nozzle 93 of the front end of the inserting portion of the hard endoscope 4 can freely be controlled.

Meanwhile, as described above, the belly portion of the patient is pierced with a plurality of trocars (pierced with 4 pieces of trocars in the drawing). In the celioscope surgical operation, an observation or a treatment is carried out by inserting a plurality of trocars into the belly portion of the patient to thereby insert various objects of an endoscope, a treatment piece and the like into the celiac coelom, and therefore, a number of trocars are pierced thereinto. When the trocar is pierced to the belly portion of the patient, inside of the celiac coelom cannot be brought into a complete hermetically closed state but more or less gas is leaked from inside of the celiac coelom. As a number of pieces of trocars pierced into the belly portion is increased, an amount of a gas leaked from inside of the celiac coelom is increased, and a pressure at inside of the celiac coelom is reduced. As a result, the celiac coelom is shriveled, and the range of operating the treatment piece 1 and the hard endoscope 4 cannot be ensured.

Although in an ordinally endoscope system, in order to replenish CO₂ gas leaked from inside of the celiac coelom, the aeroperitonia apparatus 5 detects that the pressure at inside of the celiac coelom is reduced and CO₂ gas is supplied newly from the aeroperitonia apparatus 5, according to the invention, as CO₂ gas supplied for compensating for a gas leaked out from inside of the celiac coelom, CO₂ gas injected from the injection nozzle 93 for cleaning the observation window 92 is utilized. From the view point of protecting the patient, the same CO₂ gas is used for the gas supplied from the aeroperitonia apparatus 5 for swelling the celiac coelom and the gas for ensuring the observation field of view, and therefore, CO₂ gas for ensuring the observation field of view can be utilized as CO₂ gas for replenishing the gas leaked out from inside of the celiac coelom.

Therefore, CO₂ gas for cleaning the observation window 92 is left to be discharged to inside of the celiac coelom without sucking CO₂ gas after having been injected to the observation window 92. Thereby, CO₂ gas injected from the injection nozzle 93 is used not only for ensuring the observation field of view of the observation window 92 but also for replenishing the gas leaked out from inside of the celiac coelom. As a result, an amount of CO₂ gas consumed can generally be restrained. Here, all of an amount of leaking CO₂ gas leaked out from inside of the celiac coelom may be replenished by CO₂ gas for cleaning the observation window 92. In that case, it is not necessary to newly supply CO₂ gas from the aeroperitonia apparatus 5.

As described above, CO₂ gas is intermittently injected from the injection nozzle 93 for cleaning the observation window 92, when the CO₂ gas is utilized as a leakage amount of CO₂ gas leaked out from inside of the celiac coelom, CO₂ gas is controlled such that the product of the injection pressure by the injection time period of CO₂ gas, that is, the amount of injecting CO₂ gas becomes equal to or smaller than the leakage amount of the gas from inside of the celiac coelom. Further, CO₂ gas can also be controlled to change the amount of injecting CO₂ gas by a kind of a treatment, a number of pieces of trocars or the like.

As explained above, by effectively utilizing CO₂ gas injected intermittently from the injection nozzle for cleaning the observation window as the gas for replenishing the gas leaked from inside of the celiac coelom, an amount of consuming CO₂ gas can be restrained. At this occasion, by controlling the amount of injecting CO₂ gas injected for cleaning the observation window to be equal to or smaller than the leakage amount of the gas leaked from inside of the celiac coelom, inside of the celiac coelom can be avoided from being brought into a high pressure state. Further, by freely controlling the injection pressure and the injection time period, the observation window can be cleaned in accordance with a mode of a treatment.

Further, although according to the above-described embodiment, the injection nozzle is provided at the front end of the hard endoscope, for example, a sheath may be provided between the trocar and the endoscope and the injection nozzle can be provided to the sheath.

Here, the amount of CO₂ gas leaked from inside of the celiac coelom is varied by a number or a kind of trocars pierced thereinto or a physical situation of the subject or the like. Hence, it is preferable to restrain the amount of CO₂ gas injected from the injection nozzle for forming the fluid curtain to be equal to or smaller than a half of the leakage amount from inside of the celiac coelom in consideration of the fact that the amount of leaking CO₂ gas is varied. In this case, although the supply amount is smaller than the leakage amount of CO₂ gas, the aeroperitonia apparatus is provided with the pressure detecting unit, and therefore, when the pressure at inside of the celiac coelom becomes equal to or smaller than a predetermined pressure, the pressure is detected, and the aeroperitonia control portion starts to supply CO₂ gas. That is, a deficient amount is compensated for by CO₂ gas from the aeroperitonia apparatus.

This application claims foreign priority from Japanese Patent Application No. 2006-127190, filed May 1, 2006, the entire disclosure of which is herein incorporated by reference.

## Claims

1. An endoscope system comprising:
a plurality of guide tubes that is pierced into a subject coelom swelled by supplying a pressurized gas;
an endoscope inserted into one of the plurality of guide tubes;
a treatment piece inserted into at least the other one of the plurality of guide tubes;
an injection nozzle that intermittently inject a cleaning gas to an observation window at a front end of an inserting portion of the endoscope so as to flow along a surface of the observation window, the cleaning gas being the same as the pressurized gas; and
a cleaning gas supply controller capable of setting an injection pressure and an injection time period of the cleaning gas under a condition that an injection amount of the cleaning gas becomes equal to or smaller than a leakage amount of the pressurized gas leaked from inside of the coelom.

2. The endoscope system according to Claim 1, further comprising a pressurized gas supply apparatus that supplies the pressurized gas, the pressurized gas supply apparatus comprising a pressure detecting unit that detects a pressure at inside of the coelom, wherein the pressure detecting unit supplies the, pressurized gas to inside of the coelom when the pressure at inside of the coelom becomes equal to or smaller than a specific pressure; and
wherein the cleaning gas supply controller controls an injection amount of the cleaning gas such that a total of a supply amount of the pressurized gas and the injection amount of the cleaning gas is equivalent to the leakage amount of the pressurized gas.

3. The endoscope system according to Claim 2, wherein the injection amount of the pressure gas is equal to or smaller than a half of the leakage amount of the pressurized gas.
